# EUROPEAN PATENT SPECIFICATION

(11) **EP 3 116 629 B1**
(45) Date of publication and mention of the grant of the patent: **16.01.2019**
(21) Application number: 15718586.9
(22) Date of filing: 10.03.2015
(51) Int. Cl.: B01D 53/58, C07C 273/04

(54) **METHOD FOR PROCESSING A GAS STREAM FROM A UREA PLANT SOLIDIFICATION UNIT**
VERFAHREN ZUR VERARBEITUNG EINES GASSTROMS AUS EINER SOLIDIFIZIERUNGSEINHEIT EINER HARNSTOFFANLAGE
PROCÉDÉ DE TRAITEMENT D'UN COURANT DE GAZ PROVENANT D'UNE UNITÉ DE SOLIDIFICATION D'INSTALLATION DE PRODUCTION D'URÉE

(30) Priority: 10.03.2014 IT MI20140371
(43) Date of publication of application: 18.01.2017
(73) Proprietor: SAIPEM S.p.A., 20097 San Donato Milanese (MI) (IT)
(72) Inventor: BRUNO, Lorenzo, I-20097 San Donato Milanese (IT); CARLESSI, Lino, I-24044 Dalmine (IT)
(74) Representative: Cernuzzi, Daniele
(86) International application number: PCT/IB2015/051738
(87) International publication number: WO 2015/136443

(56) References cited:
- EP-A1- 2 386 346
- EP-A2- 0 393 723
- WO-A1-99/01205
- DE-A1-102007 022 462
- FR-A5- 2 140 970
- US-A1- 2013 230 443
- US-B1- 7 618 600

## Description

### TECHNICAL FIELD

The present invention relates to a recovery method for processing a gas stream from a solidification unit of a urea plant, recovering from said gas stream components to reuse in the same recovery method and/or in the urea plant.

### BACKGROUND ART

As is known, urea is produced on an industrial scale via processes based on the reaction, under high-temperature and high-pressure conditions, between carbon dioxide and ammonia to form ammonium carbamate, and the subsequent decomposition reaction of the ammonium carbamate to provide urea and water. In a typical urea production plant (urea plant), these processes are generally carried out in a urea synthesis reactor; the aqueous urea solution produced in the synthesis reactor is then progressively concentrated, with recover of unconverted reagents, in one or more recovery sections, for example in a high-pressure section, a medium-pressure section and a low-pressure section; finally, the urea is solidified in a solidification section, which normally includes a granulator or a prilling tower.

In particular, the urea leaving the synthesis reactor is subsequently concentrated to values approximately above 95% by weight (typically 96-99. 7%) before being sent to the solidification section (granulator or prilling tower) to obtain the final commercial product, in the form of urea granules or prills.

Normally, the urea sent to the granulator or prilling tower is in a liquid state and solidifies by means of a hot stream of ambient air.

When the air used for solidification leaves the solidification section, it contains urea powder and ammonia released by the liquid urea during the cooling and solidification process.

Before releasing this air into the atmosphere, it is therefore opportune to purify it by removing the urea powder and ammonia it contains.

After having removed the urea powder, usually in a first scrubber where an aqueous solution of liquid urea circulates, it is known to remove the ammonia in a second scrubber, where a solution containing ammonia salts circulates.

For example, it is known to circulate a solution containing ammonium sulphate and ammonium bisulphate in the ammonia removal scrubber; the ammonia in the air, reacting with the ammonium bisulphate, forms ammonium sulphate and the purified air may be released into the atmosphere, while the solution of ammonium sulphate is recirculated in the scrubber to continue the treatment and, in part, removed as a by-product. In order to maintain the correct ratio between ammonium sulphate and ammonium bisulphate in the recirculating solution, it is necessary to add sulphuric acid, keeping the pH of the solution in the range 0 to 7. The by-product obtained is a solution of ammonium sulphate. The problem of using the solution of ammonium sulphate obtained arises; in fact, ammonium sulphate is a reaction by-product of little commercial interest, hygroscopic if water is removed and therefore difficult to process from the chemical-physical viewpoint. Furthermore, sulphuric acid, with the associated costs of provisioning and management, is consumed to top up the ammonium bisulphate.

In the method mentioned herein, as with other similar ones, it is therefore necessary to replenish reactants (with consequent provisioning and management costs) and the problem still arises of using the by-products, of little commercial interest.

DE 102007022462 A1 discloses a method for the treatment of an ammonium carbonate solution, wherein the ammonium carbonate solution coming from a scrubber is fed to a distillation column. Ammonia-depleted wash water is recovered from the distillation column and recirculated to the scrubber. The scrubber requires however also to be fed with fresh water. Incidentally, the wash water may include small amount of metal salts, which have to be removed.

### DISCLOSURE OF INVENTION

One object of the present invention is to provide a recovery method for processing a gas stream from a solidification unit of a urea plant, in particular for removing ammonia from said gas stream, without the described drawbacks of the known art.

In particular, an object of the invention is to provide a recovery method that enables efficiently recovering and reusing each component used or produced in the recovery method and/or in the urea plant.

The present invention therefore relates to a recovery method for processing a gas stream from a solidification unit of a urea plant as defined in claim 1.

Additional preferred features of the invention are indicated in the dependent claims.

The method of the invention allows avoiding the formation of any by-product, as each component is recovered and reused; furthermore, there is not even a requirement to add reactants from the outside, because the system and method only use components already present or produced.

### BRIEF DESCRIPTION OF THE DRAWINGS

Further characteristics and advantages of the present invention will become clear from the description of the following non-limitative embodiments, with reference to the figures in the accompanying drawings, in which:
- Figure 1 is a schematic view of a first embodiment of a recovery system which can be used in the method for processing a gas stream from a solidification unit of a urea plant in accordance with the invention;
- Figure 2 is a schematic view of a second embodiment of a recovery system which can be used in the method in accordance with the invention;
- Figure 3 is a schematic view of a third embodiment of a recovery system which can be used in the method in accordance with the invention;
- Figure 4 is a schematic view of a fourth embodiment of a recovery system which can be used in the method in accordance with the invention;
- Figure 5 is a schematic view of a fifth embodiment of a recovery system which can be used in the method in accordance with the invention;
- Figure 6 is a schematic view of a sixth embodiment of a recovery system which can be used in the method in accordance with the invention;
- Figure 7 is a schematic view of a seventh embodiment of a recovery system which can be used in the method in accordance with the invention.

### BEST MODE FOR CARRYING OUT THE INVENTION

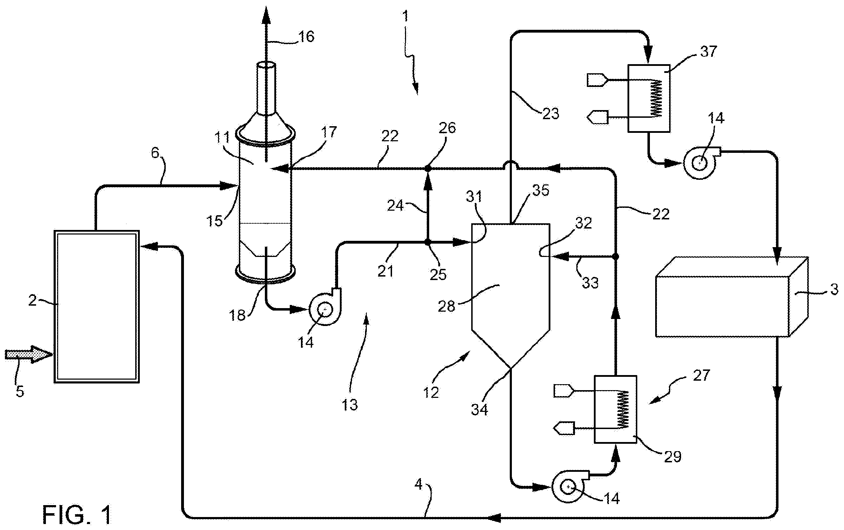

Figure 1 schematically shows a recovery system 1 for processing a gas stream leaving a solidification unit 2 of a urea plant 3 (i.e. a urea production plant) and recovering components from said gas stream to reuse in the recovery system 1 and in the urea plant 3.

The urea plant 3 is, in itself, essentially known and shall therefore be neither described nor illustrated in detail. In general terms, the urea plant 3 comprises: a urea synthesis reactor where the reaction of synthesis of urea from ammonia and carbon dioxide takes place; recovery sections (for example, in particular a high-pressure section, a medium-pressure section and a low-pressure section), in which the urea solution produced in the synthesis reactor is progressively concentrated, with the removal of water and unreacted ammonia and carbon dioxide, and recirculation of the recovered components; and a vacuum section equipped with a vacuum system and connected to a waste-water treatment section.

The urea plant 3 is connected via a urea feed line 4 to the solidification unit 2, which includes, for example, a prilling tower (as schematically shown in Figure 1) or a granulator, to which the molten urea produced in the urea plant 3 is sent to obtain solid urea in granules or prills.

The solidification unit 2 is fed with a stream of cooling air, schematically represented by the arrow 5 in Figure 1, which enters the solidification unit 2 through suitable air inlet openings, for the purpose of solidifying the molten urea.

From the solidification unit 2 a gas stream exits that is basically formed by the cooling air used in the solidification unit 2 and contains urea powder and ammonia (released by the urea during the cooling and solidification process).

After removal of the urea powder (in a suitable unit, for example a scrubber, known and in itself not relevant to the present invention and therefore neither shown nor described for simplicity), the gas stream leaving the solidification unit 2 (intended, here and hereinafter, as the gas stream leaving the solidification unit 2 and purified of urea powder), is sent to the recovery system 1 through a gas line 6 that operationally connects the solidification unit 2 to the recovery system 1.

The recovery system 1 comprises a scrubber 11 connected to the solidification unit 2 to process the gas stream from the solidification unit 2, a heat treating unit 12 operating on a recovery solution extracted by the scrubber 11, and a circuit 13, equipped with circulation pumps 14, which connects the scrubber 11, the heat treating unit 12 and the urea plant 3.

The scrubber 11 has a gas inlet 15 connected to the gas line 6, a gas outlet 16 (connected to a chimney for example), a reactant inlet 17 and a bottom outlet 18.

The function of the scrubber 11 is to remove ammonia from the gas stream leaving the solidification unit 2; the removal of ammonia in the scrubber 11 takes place through a reaction with at least one salt of a metal capable of forming ammonia complexes, as will be described in greater detail hereinafter.

The heat treating unit 12 is connected in a loop with the scrubber 11 via an inlet line 21 and a return line 22. In particular, the heat treating unit 12 is connected to the bottom outlet 18 of the scrubber 11 by the inlet line 21, to receive the recovery solution produced in the scrubber 11 and subject it to a heat treatment in a controlled temperature range, and to the reactant inlet 17 of the scrubber 11 by the return line 22, to return a reactant composition, produced in the heat treating unit 12 and containing at least one ammonia salt, to the scrubber 11.

The heat treating unit 12 is also connected to the urea plant 3 by an ammonia line 23.

The circuit 13 thus comprises: the inlet line 21 and the return line 22, which connect the heat treating unit 12 and the scrubber 11; and the ammonia line 23, which connects the heat treating unit 12 to the urea plant 3.

In addition, the circuit 13 comprises a bypass line 24 that connects the inlet line 21 to the return line 22, excluding the heat treating unit 12 (i.e. it connects a branch point 25 of the inlet line 21 located upstream of the heat treating unit 12 to an inflow point 26 of the return line 22 located downstream of the heat treating unit 12).

The heat treating unit 12 is equipped with heating means 27 to heat the recovery solution up to a controlled temperature, or within a controlled temperature range, such as to separate the ammonia, to be reused in the urea plant 3, and produce a molten reactant composition, containing at least one ammonia salt, to be recirculated to the scrubber 11 and used to remove ammonia from the gas stream coming from the solidification unit 2.

In the embodiment in Figure 1, the heating means 27 of the heat treating unit 12 comprise an oven 28, constituted, for example (but not necessarily), by a crucible, possibly lined with a refractory material, and a heat exchanger 29, in particular an indirect-contact heat exchanger in which a heating fluid circulates, this fluid being constituted, for example, by diathermic oil, steam (for example, medium-pressure steam taken from the medium-pressure section of the urea plant 3), combustion fumes, or some other fluid.

The oven 28 has a main inlet 31 connected to the inlet line 21 and therefore to the bottom outlet 18 of the scrubber 11, a secondary inlet 32 connected to the heat exchanger 29 via a service line 33, a bottom outlet 34 connected to the return line 22, and a top outlet 35 connected to the ammonia line 23.

In particular, the heat exchanger 29 is located along the return line 22 and is connected in a loop with the oven 28 via service line 33, which branches from the return line 22 downstream of the heat exchanger 29 and connects the heat exchanger 29 to the secondary inlet 32 of the oven 28.

The ammonia line 23 is equipped with a condensation unit 37, for example one fed with cold water, which also acts as a vacuum system for extracting gas streams from the oven 28 (clearly, a different vacuum system or a pressurized extraction system could also be provided for extraction).

Circulation pumps 14 are positioned, for example, on the inlet line 21, the return line 22 (in particular, between the oven 28 and the heat exchanger 29) and the ammonia line 23.

In use, the solidification unit 2 receives molten urea from the urea plant 3 through the urea feed line 4 and is fed with cooling air 5. From the solidification unit 2 solid urea (in granules or prills) is collected and a gas stream exits that is basically formed by the cooling air used in the solidification unit 2 and containing ammonia, which is sent to the recovery system 1 (more precisely, to the scrubber 11) through the gas line 6.

The gas stream leaving the solidification unit 2 (after being preliminarily treated for the removal of urea powder), is processed in the scrubber 11 to remove the ammonia it contains via a reaction with at least one (molten) salt containing a metal capable of forming an ammonia complex.

A complex is a chemical compound in which a central atom or ion binds with a number (even higher than the oxidation number of the central atom/ion) of other chemical species (atoms, ions or molecules), known as ligands and which surround the central atom/ion. In general, the central atom/ion and the ligands form an ion known as a "complex ion", the salt of which is known as a "complex compound".

The ammonia complex of the invention is a compound formed by a central atom of a metal, specifically a metal ion, and by ligands constituted by ammonia molecules (NH₃).

In accordance with the invention, the scrubber 11 is fed, through the return line 22, with a reactant composition containing at least one molten salt of a metal capable of forming ammonia complexes; the gas stream produced in the solidification unit 2 is freed from ammonia inside the scrubber 11 and may be discharged into the atmosphere (through the gas outlet 16); the ammonia is captured by the metal contained in the salt of the reactant composition, forming an ammonia complex; and a recovery solution containing the ammonia complex in an aqueous solution (i.e. the ion complex formed by the metal and by a number of ammonia molecules) is recovered from the scrubber 11 and sent to the heat treating unit 12.

In the heat treating unit 12, the recovery solution is heated to a controlled temperature to degrade the ammonia complex and separate the ammonia, to be reused in the urea plant 3, and produce at least one molten salt containing the metal (metal ion) capable of forming ammonia complexes, to recirculate to the scrubber 11 and use for removing ammonia from the gas stream coming from the solidification unit 2.

The temperature at which the heat treating unit 12 operates is such as to cause degradation of the ammonia complex contained in the recovery solution to give gaseous ammonia and molten salt, but avoid (or limit) further degradation of the salt (in particular of its anion) to give gaseous compounds that would pollute the recovered ammonia.

The scrubber 11 is fed with a salt of a metal selected in the group consisting of: Cr (chromium) and Ag (silver); or a metal of the Irving-Williams series: Mn (manganese), Fe (iron), Co (cobalt), Ni (nickel), Cu (copper), or Zn (zinc). Advantageously (but not necessarily), the salts used are sulphates, phosphates or nitrates.

The aqueous solution of the ammonia complex, i.e. containing the complex ion formed by the metal and by a number of ammonia molecules, and the ion (anion) of the initial salt (introduced into the scrubber 11 to remove the ammonia), constitutes the recovery solution leaving the scrubber 11 and which is taken from the bottom outlet 18 of the scrubber 11 and sent through the inlet line 21 to the heat treating unit 12.

The pH of the recovery solution is generally basic (for example, between approximately 7 and 9).

The heat treating unit 12 serves to obtain ammonia (in the gaseous phase, i.e. ammonia vapours) from the recovery solution containing the ammonia complex and coming from the scrubber 11, the ammonia then being sent through the ammonia line 23 to the urea plant 3 for the production of urea, and the reactant composition, containing the salt of the metal capable of forming ammonia complexes, which is recirculated to the scrubber 11 through the return line 22 and reused in the scrubber 11 for removing ammonia from the gas stream coming from the solidification unit 2.

To recover the ammonia complex (rich in ammonia) from the recovery solution coming from the scrubber 11, the ammonia complex is heated in the heat treating unit 12 to a temperature between approximately 200°C and approximately 350°C.

At these temperatures, gaseous ammonia is released with the formation of molten salt containing the metal ion. Schematically, the following reaction takes place in the scrubber 11:

(I) M^{m+} + n NH₃ → [M(NH₃)ₙ]^{m+}

The thermal degradation of the ammonia complex produced in the scrubber 11 takes place in the heat treating unit 12, via the following reaction:

(II) [M(NH₃)ₙ]^{m+} + heat → M^{m+} + n NH₃

This reaction starts to occur very rapidly at temperatures from 200°C upwards.

However, for temperatures higher than 350°C, other undesired decomposition reactions may also happen; for example, if sulphates are used, the following reactions may occur:

(III) SO₄²⁻ + H₂O + heat → 2OH⁻ + SO₃

(IV) 2SO₃ + heat → O₂ + 2SO₂

Since these reactions (and similar reactions that occur with other salts, such as phosphates, nitrates, etc.) may result in having pollutants mixed with the ammonia vapours, which complicates the subsequent recovery and use of the ammonia in the urea plant 3, it is preferable that the heat treating unit 12 operates within a temperature range of 200-350°C.

For example, if a copper sulphate is used as the salt for forming the ammonia complexes, the capture of the ammonia in the scrubber 11 takes place according to the following reaction:

(V) Cu²⁺ + SO₄²⁻ + 4NH₃ + 2H₂O → [Cu(NH₃)₄(H₂O)₂]²⁺ + SO₄²⁻

The copper ammonia complex may then be degraded according to the following reaction:

(VI) [Cu(NH₃)₄(H₂O)₂]²⁺ + SO₄²⁻ → Cu²⁺ + SO₄²⁻ + 4(NH₃)_{gas} + 2H₂O

In this case, the recovery solution (leaving the scrubber 11) is an aqueous solution containing the copper ammonia complex; the reactant composition formed in the heat treating unit 12 and recovered in the scrubber 11 contains copper sulphate (as the salt for forming the ammonia complex).

The heat necessary for the thermal degradation of the ammonia complex is provided in the heat treating unit 12.

A recycle portion of the recovery solution (ammonia complex solution) produced in the scrubber 11 is recirculated to the scrubber 11 through the bypass line 24; the remainder is sent to the heat treating unit 12 and, in particular, to the oven 28, where the ammonia is separated to obtain the reactant composition containing the salt of the metal capable of forming the ammonia complex.

Therefore, with regard to the reactants necessary for removing the ammonia, the scrubber 11 is fed with the reactant composition produced in the heat treating unit 12, with the addition of a portion (recycle portion) of the recovery solution; the addition of fresh components is not required.

The gaseous ammonia produced in the heat treating unit 12 is extracted from the oven 28 through the top outlet 35, and, after running through the ammonia line 23, is condensed in the condensation unit 37 and finally sent to the urea plant 3, to be reused.

With the layout shown in Figure 1, given that the recovery solution coming from the scrubber 11 is an aqueous solution that is sent directly to the oven 28, it is also necessary to provide heat for the evaporation of the water present, which will then be found in the ammonia vapours leaving from the top outlet 35 of the oven 28. Clearly, the higher the concentration of the recovery solution, the lesser the heat needed to evaporate the water contained therein.

The recovery solution coming from the scrubber 11 may be brought to the operating temperature for degradation of the ammonia complex present therein (200-350°C) and be kept molten by adjusting the temperature in various ways, other than that described above.

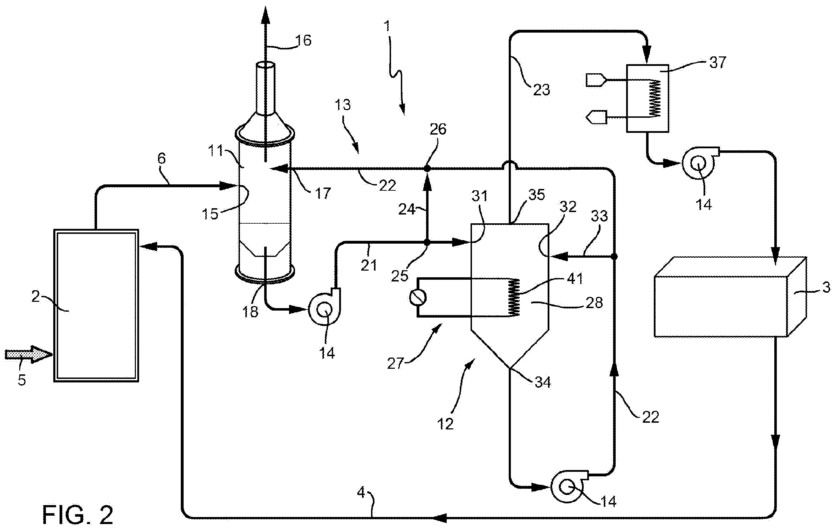

For example, in Figures 2-7, where details similar or identical to those already described are indicated with the same reference numerals, several embodiments of the invention are shown, in which the recovery system 1 has different configurations and includes various types of heat treating units 12.

In all the plant configurations shown, a reactant composition (fed to the scrubber 11), containing at least one salt (for example, a sulphate, a phosphate, a nitrate, etc.) of a metal capable of forming ammonia complexes, and a recovery solution (extracted from the scrubber 11), containing the complex ion formed by the metal and by ammonia molecules, are used.

In the embodiment in Figure 2, the heat treating unit 12 comprises an electric oven 28 with an electric resistance.

In particular, the oven 28 includes at least one electric resistance 41 placed inside the oven 28 and, for example, immersed in the molten bath that forms in the oven 28.

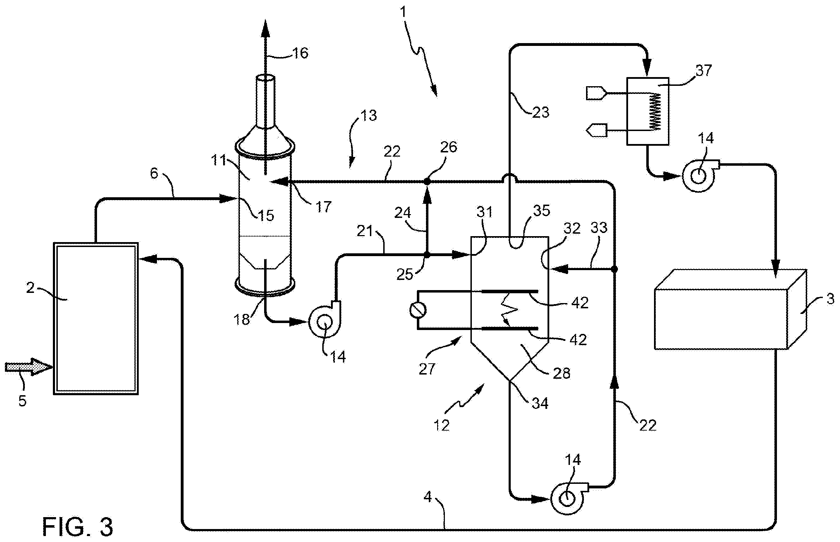

In the embodiment in Figure 3, the heat treating unit 12 comprises an electric oven 28 with immersed electrodes.

In particular, the oven 28 includes at least one pair of electrodes 42 (for example in graphite or equivalent materials), placed in oven 28 and immersed in the molten bath contained in the oven 28.

This embodiment is particularly cost-effective from the investment cost viewpoint and significantly reduces problems linked with materials corrosion.

In Figure 3, a pump 14 (which also provides for the recirculation of the reactant composition to the oven 28 and to the scrubber 11) is used for extraction of the reactant composition (containing the molten salt) from the oven 28.

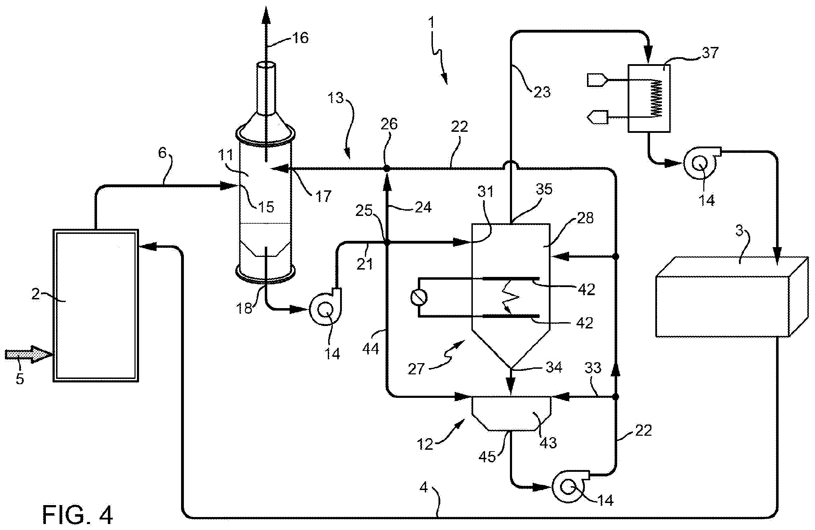

Also in the embodiment in Figure 4 the heat treating unit 12 comprises an electric oven 28 with immersed electrodes 42. The oven 28 is connected to a mixer 43 located, for example, underneath the bottom outlet 34 of the oven 28.

Similarly to that previously described, the oven 28 separates the ammonia and produces the molten salt, which in this case, however, before being recirculated to the scrubber 11, is mixed in the mixer 43 with a portion of the recovery solution coming from the scrubber 11, thereby incorporating an aqueous component (solution).

The molten salt produced in the oven 28 is collected in the mixer 43, where, for example, it falls under gravity; an auxiliary line 44, which runs from the inlet line 21 upstream of the oven 28 (for example, from branch point 25), conveys a portion of the recovery solution leaving the scrubber 11 to the mixer 43.

The return line 22, equipped with a pump 14, connects an outlet 45 of the mixer 43 to the reactant inlet 17 of the scrubber 11.

The service line 33 runs from the return line 22 and recirculates a portion of the reactant composition circulating in the return line 22 (which in this case is, as indicated above, the aqueous solution formed in the mixer 43). In this way, the pump 14 located on the return line 22 operates on the aqueous solution produced in the mixer 43 (and not on the ammonia complex mixture leaving the oven 28).

Also in this case, the bypass line 24, which runs from the inlet line 21 (for example from branch point 25) and merges into the return line 22 (in particular, at inflow point 26), recirculates a recycle portion of the recovery solution coming from the scrubber 11 directly back to the scrubber 11, without passing through the heat treating unit 12.

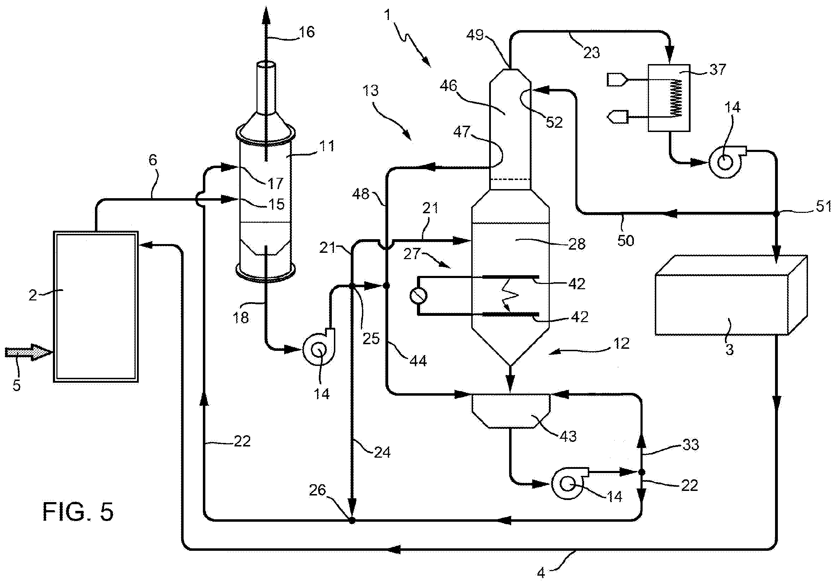

In the embodiment in Figure 5, the heat treating unit 12 still includes an electric oven 28 with immersed electrodes, associated with a mixer 43.

With respect to the previous embodiment in Figure 4, the heat treating unit 12 includes also a distillation column 46, placed, in particular, at the top of the oven 28 to separate the water from the ammonia in the gas stream leaving the oven 28, before the ammonia is condensed and sent to the urea plant 3.

The distillation column 46 receives the gaseous phase produced in the oven 28 (basically formed of ammonia and water) and separates the water, which flows out from a bottom outlet 47 of the distillation column 46 and, through a makeup line 48, flows into the auxiliary line 44 to feed the mixer 43, from the ammonia, which flows out from a top outlet 49 of the distillation column 46 and into the ammonia line 23.

A recirculation line 50 connects a branch point 51 of the ammonia line 23, located downstream of the condensation unit 37, to a top inlet 52 of the distillation column 46, to recirculate liquid ammonia to the distillation column 46.

The distillation column 46 does not need a bottom reboiler, as hot ammonia vapours coming from the oven 28 are used.

The mixer 43 is fed, via the auxiliary line 44 that runs from the inlet line 21 upstream of the oven 28 (for example, from branch point 25), with a portion of the recovery solution leaving the scrubber 11, with the addition of water extracted from the distillation column 46 and which flows into auxiliary line 44 through the makeup line 48.

As in the other embodiments, the bypass line 24, which connects the inlet line 21 to the return line 22 (connecting, in particular, branch point 25 to inflow point 26), recirculates a recycle portion of the recovery solution coming from the scrubber 11 directly back to the scrubber 11, without passing through the heat treating unit 12.

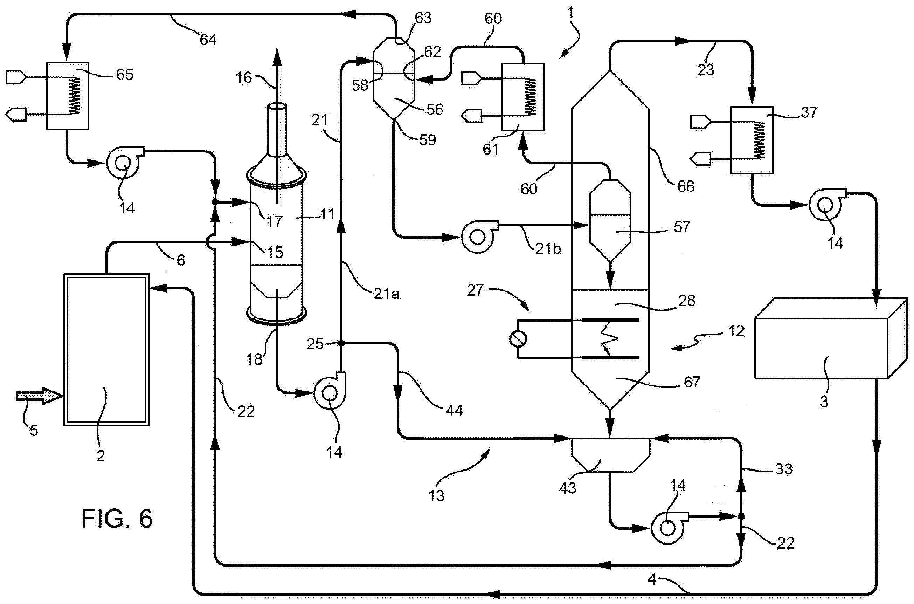

In the embodiment in Figure 6, the heat treating unit 12 still includes an oven 28, for example an electric oven with immersed electrodes, and a mixer 43. The recovery system 1 also includes a crystallizer 56 (or other type of concentrator) located along the inlet line 21 between the scrubber 11 and the oven 28, and a hydrocyclone 57 (or other type of solid/liquid separator) positioned, for example, above the oven 28.

Before being sent to the oven 28, the recovery solution coming from the scrubber 11 is sent to the crystallizer 56 to separate the water from the recovery solution, so as to supply the oven 28 with the ammonia complex in a crystallized (or at least concentrated) form.

The recovery solution extracted from the scrubber 11 is thus sent to the crystallizer 56 through a first section 21a of the inlet line 21 (which connects the bottom outlet 18 of the scrubber 11 to a first inlet 58 of the crystallizer 56); a second section 21b of the inlet line 21 then connects a bottom outlet 59 of the crystallizer 56 to the hydrocyclone 57.

Heat is provided inside the crystallizer 56 to deposit slurry containing an aqueous solution and solids on the bottom of an internal crystallization chamber of the crystallizer 56; this slurry is sent, via section 21b, to the hydrocyclone 57, where the solids are separated from the solution. The solids (with traces of water) are sent to the oven 28; the solution is sent through a line 60 to a reboiler 61 (or other heat exchanger) and subsequently to a second inlet 62 of the crystallizer 56, to provide heat for the crystallization.

A vapour phase taken from the crystallizer 56, specifically from a top outlet 63 of the crystallizer 56, runs through a vapour line 64 and, after optionally passing through a heat exchanger 65, is fed back into the scrubber 11, for example, by flowing into the return line 22.

To avoid handling the solids, the hydrocyclone 57 is advantageously located on top of the oven 28, or inside the oven 28 or a shell 66 that also contains the oven 28, over a bottom zone 67 of the oven 28 occupied by the molten bath that forms in the oven 28: in this way, the solids separated in the hydrocyclone 57 are transferred by falling into the oven 28 where fusion of the salt takes place. In addition, it is also possible to recover part of the heat of the ammonia vapours produced in the oven 28 to keep the hydrocyclone 57, and the solution leaving it, hot.

Optionally, instead of being sent to the crystallizer 56, part of the recovery solution is sent to the mixer 43 through the auxiliary line 44, which runs from the inlet line 21 upstream of the crystallizer 56 (in particular, from branch point 25).

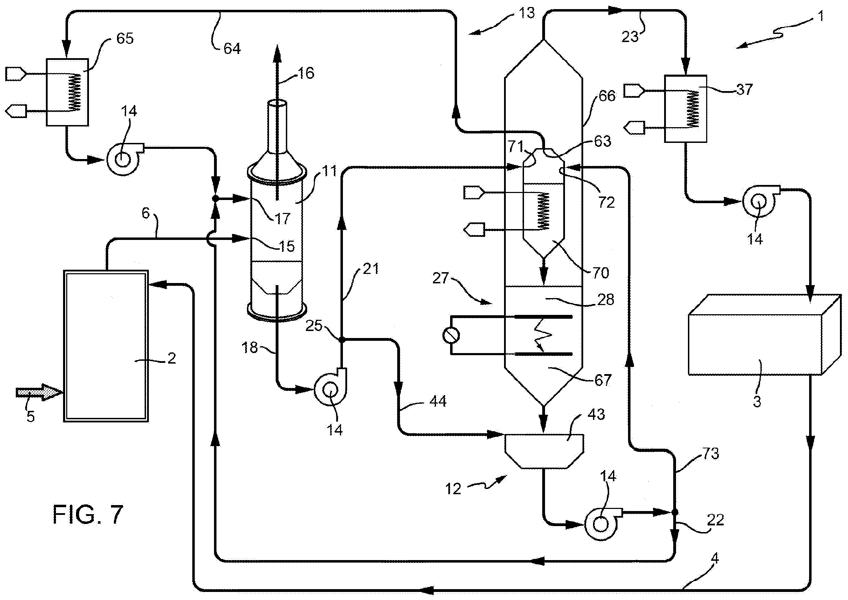

Similarly, in the embodiment in Figure 7, the recovery solution coming from the scrubber 11 is concentrated, via the removal of water, before being sent to the oven 28.

In this case, the recovery system 1 comprises an evaporator 70 (concentrator), which receives the recovery solution and separates water from the recovery solution, so as to supply the oven 28 with a concentrated product containing the ammonia complex.

A portion of the recovery solution extracted by the scrubber 11 is sent to the evaporator 70 through the inlet line 21 (which connects the bottom outlet 18 of the scrubber 11 to a first inlet 71 of the evaporator 70).

The remainder of the recovery solution is instead sent to the mixer 43 through the auxiliary line 44, which runs from the inlet line 21 upstream of the evaporator 70 (in particular, from branch point 25).

The evaporator 70 is heated, for example via low-pressure steam, to evaporate water and form the concentrate (containing the ammonia complex) that is discharged into the oven 28.

Advantageously, the evaporator 70 is located on top of the oven 28, or inside the oven 28 or the shell 66 that also contains the oven 28, over the bottom zone 67 of the oven 28 occupied by the molten bath that forms in the oven 28: in this way, the concentrate formed in the evaporator 70 may be discharged directly into the oven 28 and it is possible to recover part of the heat of the ammonia vapours produced in the oven 28 to heat the evaporator 70.

Advantageously, the evaporator 70 has a second inlet 72 connected to a service line 73 that runs from the return line 22 to transfer a portion of the reactant composition circulating in the return line 22 (solution formed in the mixer 43 and containing the mixture of molten salt produced in the oven 28 and a portion of the recovery solution) to the evaporator 70.

A vapour phase taken from the evaporator 70, specifically from a top outlet 63 of the evaporator 70, runs through a vapour line 64 and, after optionally passing through a heat exchanger 65, is fed back into the scrubber 11, for example, by flowing into the return line 22.

In all of the above described embodiments, the gaseous ammonia produced in the heat treating unit 12 is condensed in the condensation unit 37 and then sent to the urea plant 3, for reuse.

Finally, it is understood that further modifications and variants may be made to the method described and illustrated herein without departing from the scope of the appended claims.

## Claims

1. A recovery method for processing a gas stream from a solidification unit (2) of a urea plant (3), in particular a cooling air stream used in the solidification unit (2) and containing ammonia; comprising a step of processing the gas stream from the solidification unit (2) in a scrubber (11) to remove from said gas stream the ammonia contained in said gas stream from the solidification unit (2) via a reaction with at least one molten salt of a metal capable of forming ammonia complexes; the scrubber (11) being fed with a reactant composition containing said at least one molten salt of a metal capable of forming ammonia complexes; the gas stream produced in the solidification unit (2) being freed from ammonia inside the scrubber (11); the ammonia being captured by the metal contained in the salt of the reactant composition, forming an ammonia complex; a recovery solution containing the ammonia complex in an aqueous solution being recovered from the scrubber (11) and sent to a heat treating unit (12); the method further comprising the steps of:
- drawing said recovery solution, containing said at least one ammonia complex, from the scrubber (11);
- heat treating said recovery solution by heating it to a controlled temperature such as to degrade said at least one ammonia complex and produce gaseous ammonia and said salt; and forming a molten or liquid reactant composition containing said salt;
- feeding at least part of the reactant composition to the scrubber (11), to remove ammonia from the gas stream from the solidification unit (2); and
- recovering the ammonia produced in the heat treating step and feeding it to the urea plant (3);
wherein the metal capable of forming ammonia complexes is a metal selected in the group consisting of: Cr (chromium), Ag (silver); Mn (manganese), Fe (iron), Co (cobalt), Ni (nickel), Cu (copper) and Zn (zinc).

2. A recovery method according to claim 1, wherein the salt is a sulphate, a phosphate or a nitrate.

3. A recovery method according to one of the preceding claims, comprising the step of condensing the ammonia produced in the heat treating step, before feeding it to the urea plant (3) .

4. A recovery method according to one of the preceding claims, comprising the step of feeding the scrubber (11) with the reactant composition produced in the heat treating step, with the addition of a recycled portion of the recovery solution and without adding fresh reactants.

5. A recovery method according to claim 4, wherein the heat treating step is performed within a temperature range of 200-350 °C.

6. A recovery method according to one of the preceding claims, comprising the step of returning a recycle portion of the recovery solution to the scrubber (11), without subjecting the recycle portion to the heat treating step.

7. A recovery method according to one of the preceding claims, comprising the step of mixing the reaction composition, produced in the heat treating step, with part of the recovery solution from the scrubber (11) and not subjected to the heat treating step.

8. A recovery method according to one of the preceding claims, comprising the step of recirculating a part of the reactant composition to the heat treating step.

9. A recovery method according to one of the preceding claims, comprising a distillation step, in which a gaseous phase, containing water and ammonia and produced in the heat treating step, is distilled to separate the water from the ammonia.

10. A recovery method according to one of the preceding claims, comprising a crystallization step to concentrate the recovery solution from the scrubber (11) prior to the heat treating step and form sludge containing an aqueous solution and solids; and a solid/liquid separation step to separate the solids, which are sent to the heat treating step, from the aqueous solution, which is fed to a reboiler (61) or other heat exchanger to supply heat to the crystallization step.

11. A recovery method according to one of the preceding claims, comprising a recovery solution evaporation step, to separate water from the recovery solution and supply the heat treating step with a concentrated product.

## Patentansprüche

1. Rückgewinnungsverfahren zum Verarbeiten eines Gasstroms von einer Verfestigungseinheit (2) einer Harnstoffanlage (3), insbesondere eines Kühlluftstroms, der in der Verfestigungseinheit (2) verwendet wird und Ammoniak enthält; umfassend einen Schritt des Verarbeitens des Gasstroms von der Verfestigungseinheit (2) in einem Nassabscheider (11), um aus dem Gasstrom den Ammoniak, der in dem Gasstrom von der Verfestigungseinheit (2) enthalten ist, mittels einer Reaktion mit mindestens einem geschmolzenen Salz eines Metalls, das in der Lage ist, Ammoniakkomplexe zu bilden, zu entfernen; wobei der Nassabscheider (11) mit einem Reaktionspartnergemisch beschickt wird, das das mindestens eine geschmolzene Salz eines Metalls enthält, das in der Lage ist, Ammoniakkomplexe zu bilden; wobei der Gasstrom, der in der Verfestigungseinheit (2) hergestellt wird, im Innern des Nassabscheiders (11) von Ammoniak befreit wird; wobei der Ammoniak von dem Metall, das in dem Salz des Reaktionspartnergemischs enthalten ist, aufgenommen wird, wobei ein Ammoniakkomplex gebildet wird; wobei eine Rückgewinnungslösung, die den Ammoniakkomplex in einer wässrigen Lösung enthält, von dem Nassabscheider (11) zurückgewonnen und zu einer Wärmebehandlungseinheit (12) geschickt wird; wobei das Verfahren überdies die folgenden Schritte umfasst:
- Ziehen der Rückgewinnungslösung, die den mindestens einen Ammoniakkomplex enthält, aus dem Nassabscheider (11);
- Wärmebehandeln der Rückgewinnungslösung mittels Erhitzen davon auf eine gesteuerte Temperatur, zum Beispiel zum Abbauen von dem mindestens einen Ammoniakkomplex und Herstellen von Ammoniakgas und dem Salz; und Bilden eines geschmolzenen oder flüssigen Reaktionspartnergemischs, das das Salz enthält;
- Fördern von mindestens einem Teil des Reaktionspartnergemischs zu dem Nassabscheider (11), um Ammoniak aus dem Gasstrom von der Verfestigungseinheit (2) zu entfernen; und
- Rückgewinnen des Ammoniaks, der in dem Wärmebehandlungsschritt hergestellt wurde, und Fördern davon zu der Harnstoffanlage (3);
wobei das Metall, das in der Lage ist, Ammoniakkomplexe zu bilden, ein Metall ist, das in der Gruppe ausgewählt ist, die aus Folgendem besteht: Cr (Chrom), Ag (Silber); Mn (Mangan), Fe (Eisen), Co (Kobalt), Ni (Nickel), Cu (Kupfer) und Zn (Zink).

2. Rückgewinnungsverfahren nach Anspruch 1, wobei das Salz ein Sulfat, ein Phosphat oder ein Nitrat ist.

3. Rückgewinnungsverfahren nach einem der vorhergehenden Ansprüche, umfassend den Schritt des Verdichtens des Ammoniaks, der in dem Wärmebehandlungsschritt hergestellt wurde, bevor er der Harnstoffanlage (3) zugeführt wird.

4. Rückgewinnungsverfahren nach einem der vorhergehenden Ansprüche, umfassend den Schritt des Beschickens des Nassabscheiders (11) mit dem Reaktionspartnergemisch, das in dem Wärmebehandlungsschritt hergestellt wurde, mit Hinzufügen eines recycelten Abschnitts der Rückgewinnungslösung und ohne Hinzufügen von frischen Reaktionspartnern.

5. Rückgewinnungsverfahren nach Anspruch 4, wobei der Wärmebehandlungsschritt innerhalb eines Temperaturbereichs von 200 - 350 °C ausgeführt wird.

6. Rückgewinnungsverfahren nach einem der vorhergehenden Ansprüche, umfassend den Schritt des Rückleitens eines recycelten Abschnitts der Rückgewinnungslösung zu dem Nassabscheider (11), ohne den recycelten Abschnitt dem Wärmebehandlungsschritt zu unterziehen.

7. Rückgewinnungsverfahren nach einem der vorhergehenden Ansprüche, umfassend den Schritt des Mischens des Reaktionsgemischs, das in dem Wärmebehandlungsschritt hergestellt wird, mit einem Teil der Rückgewinnungslösung von dem Nassabscheider (11), und nicht dem Wärmebehandlungsschritt unterzogen wird.

8. Rückgewinnungsverfahren nach einem der vorhergehenden Ansprüche, umfassend den Schritt des Wiederzuführens eines Teils des Reaktionspartnergemischs zu dem Wärmebehandlungsschritt.

9. Rückgewinnungsverfahren nach einem der vorhergehenden Ansprüche, umfassend einen Destillationsschritt, in dem eine gasförmige Phase, die Wasser und Ammoniak enthält und in dem Wärmebehandlungsschritt hergestellt wird, destilliert wird, um das Wasser von dem Ammoniak zu trennen.

10. Rückgewinnungsverfahren nach einem der vorhergehenden Ansprüche, das Folgendes umfasst: einen Kristallisierungsschritt, um die Rückgewinnungslösung von dem Nassabscheider (11) vor dem Wärmebehandlungsschritt zu konzentrieren und einen Schlamm zu bilden, der eine wässrige Lösung und Feststoffe enthält; und einen Feststoff/Flüssigkeit-Trennschritt, um die Feststoffe, die zu dem Wärmebehandlungsschritt geschickt werden, von der wässrigen Lösung, die zu einem Wiederverdampfer (61) oder anderen Wärmetauscher gefördert wird, um Wärme für den Kristallisierungsschritt zu liefern, zu trennen.

11. Rückgewinnungsverfahren nach einem der vorhergehenden Ansprüche, umfassend einen Rückgewinnungslösung-Verdampfungsschritt, um Wasser von der Rückgewinnungslösung zu trennen und dem Wärmebehandlungsschritt ein konzentriertes Produkt zu liefern.

## Revendications

1. Procédé de récupération pour traiter un courant gazeux provenant d'une unité de solidification (2) d'une installation de production d'urée (3), en particulier un courant d'air de refroidissement utilisé dans l'unité de solidification (2) et contenant de l'ammoniac ; comprenant une étape de traitement du courant gazeux provenant de l'unité de solidification (2) dans un laveur (11) pour éliminer dudit courant gazeux l'ammoniac contenu dans ledit courant gazeux provenant de l'unité de solidification (2) via une réaction avec au moins un sel fondu d'un métal dans une solution aqueuse capable de former des complexes d'ammoniac ; le laveur (11) étant alimenté avec une composition de réactif contenant ledit au moins un sel fondu d'un métal capable de former des complexes d'ammoniac ; le courant gazeux produit dans l'unité de solidification (2) étant débarrassé de l'ammoniac à l'intérieur du laveur (11) ; l'ammoniac étant capturé par le métal contenu dans le sel de la composition de réactif, en formant un complexe d'ammoniac ; une solution de récupération contenant le complexe d'ammoniac dans une solution aqueuse étant récupérée à partir du laveur (11) et envoyée vers une unité de traitement thermique (12) ; le procédé comprenant en outre les étapes de :
- aspiration de ladite solution de récupération, contenant ledit au moins un complexe d'ammoniac, à partir du laveur (11) ;
- traitement thermique de ladite solution de récupération en la chauffant à une température contrôlée de façon à dégrader ledit au moins un complexe d'ammoniac et produire de l'ammoniac gazeux et ledit sel ; et formation d'une composition de réactif fondue ou liquide contenant ledit sel ;
- introduction d'au moins une partie de la composition de réactif dans le laveur (11), pour éliminer l'ammoniac du courant gazeux provenant de l'unité de solidification (2) ; et
- récupération de l'ammoniac produit dans l'étape de traitement thermique et son introduction dans l'installation de production d'urée (3) ;
dans lequel le métal capable de former des complexes d'ammoniac est un métal sélectionné dans le groupe consistant en : Cr (chrome), Ag (argent) ; Mn (manganèse), Fe (fer), Co (cobalt), Ni (nickel), Cu (cuivre) et Zn (zinc).

2. Procédé de récupération selon la revendication 1, dans lequel le sel est un sulfate, un phosphate ou un nitrate.

3. Procédé de récupération selon l'une des revendications précédentes, comprenant l'étape de condensation de l'ammoniac produit dans l'étape de traitement thermique, avant son introduction dans l'installation de production d'urée (3).

4. Procédé de récupération selon l'une des revendications précédentes, comprenant l'étape d'alimentation du laveur (11) avec la composition de réactif produite dans l'étape de traitement thermique, avec l'ajout d'une portion recyclée de la solution de récupération et sans ajouter de réactifs frais.

5. Procédé de récupération selon la revendication 4, dans lequel l'étape de traitement thermique est effectuée dans une plage de température allant de 200 à 350 °C.

6. Procédé de récupération selon l'une des revendications précédentes, comprenant l'étape de renvoi d'une portion de recyclage de la solution de récupération au laveur (11), sans soumettre la portion de recyclage à l'étape de traitement thermique.

7. Procédé de récupération selon l'une des revendications précédentes, comprenant l'étape de mélange de la composition de réaction, produite dans l'étape de traitement thermique, avec une partie de la solution de récupération provenant du laveur (11) et non soumise à l'étape de traitement thermique.

8. Procédé de récupération selon l'une des revendications précédentes, comprenant l'étape de remise en circulation d'une partie de la composition de réactif vers l'étape de traitement thermique.

9. Procédé de récupération selon l'une des revendications précédentes, comprenant une étape de distillation, dans laquelle une phase gazeuse, contenant de l'eau et de l'ammoniac et produite dans l'étape de traitement thermique, est distillée pour séparer l'eau de l'ammoniac.

10. Procédé de récupération selon l'une des revendications précédentes, comprenant une étape de cristallisation pour concentrer la solution de récupération provenant du laveur (11) avant l'étape de traitement thermique et former une boue contenant une solution aqueuse et des solides ; et une étape de séparation solide/liquide pour séparer les solides, qui sont envoyés vers l'étape de traitement thermique, de la solution aqueuse, qui est introduite dans un rebouilleur (61) ou un autre échangeur de chaleur pour fournir de la chaleur à l'étape de cristallisation.

11. Procédé de récupération selon l'une des revendications précédentes, comprenant une étape d'évaporation de solution de récupération, pour séparer l'eau de la solution de récupération et fournir à l'étape de traitement thermique un produit concentré.
